(19) Europäisches Patentamt / European Patent Office / Office européen des brevets

(11) **EP 3 235 427 A1**

(12) **EUROPEAN PATENT APPLICATION**

(43) Date of publication:
**25.10.2017 Bulletin 2017/43**

(51) Int Cl.:
*A61B 5/055* (2006.01)   *A61B 5/00* (2006.01)
*G06F 19/00* (2011.01)

(21) Application number: **16166489.1**

(22) Date of filing: **21.04.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **CodeBox Computerdienste GmbH 70184 Stuttgart (DE)**

(72) Inventors:
• **Mersmann, Jochen**
 **70184 Stuttgart (DE)**

• **Jirsa, Victor**
 **13400 Aubagne (FR)**

(74) Representative: **Pfenning, Meinig & Partner mbB Patent- und Rechtsanwälte Joachimsthaler Straße 10-12 10719 Berlin (DE)**

Remarks:
Claims 16 to 21 are deemed to be abandoned due to non-payment of the claims fees (Rule 45(3) EPC).

(54) **METHOD AND SYSTEM FOR ESTIMATING A LOCATION OF AN EPILEPTOGENIC ZONE OF A MAMMALIAN BRAIN**

(57)     The invention relates to a method for estimating a location of an epileptogenic zone of a mammalian brain, a system for performing a method for estimating a location of an epileptogenic zone of a mammalian brain as well as a computer-readable medium including a set of instructions for estimating a location of an epileptogenic zone of a mammalian brain.

Fig. 1

**Description**

This application discloses a method for estimating a location of an epileptogenic zone of a mammalian brain optimizing the placement of stereotactic electrodes, a system for performing said method, and a computer-readable medium containing instructions for performing such a method, as outlined by the claims and the content of this description.

Personalized medicine proposes the customization of healthcare with medical decisions, practices, and products being tailored to the individual patient. Inter-individual variability between different patients has clear effects upon the responsiveness to treatment approaches.

Patients suffering from brain seizures, such as epileptic seizures, may receive drug treatment. However, in certain cases, a possible treatment for patients having, for example, a drug-resistant form of seizures is the surgical resection of the epileptogenic zone. An epileptogenic zone is a localized region or network within the patient's brain where seizures arise, before recruiting secondary brain networks called the propagation zone. The propagation zone includes areas of the patient's brain that are affected by, for example, a seizure arising in the epileptogenic zone, the seizure then spreading into the areas encompassing the propagation zone.

Traditionally, as a part of the standard pre-surgical evaluation, stereotactic electroencephalograms (SEEG) are used to help correctly identify the epileptogenic zone. Alternative imaging techniques to the SEEG are structural magnetic resonance imaging (MRI), classical electroencephalograms (EEG) or magnetoencephalograms (MEG), computed tomography (CT) scans, and single-photon emission computed tomography (SPECT). All of these methods have been used to help professionals in outlining the epileptogenic zone. Recently, diffusion MRI (dMRI) and other methods reflecting the connectivity between different brain regions have been used as well in analyzing seizures.

Since the surgical resection of an assumed epileptogenic zone is irreversible and may cause severe brain damage, it would be helpful to minimize the risk by optimizing the identification of the epileptogenic zone.

Furthermore, the implantation of electrodes into the mammalian brain for recording SEEGs is a high-risk procedure and should only be performed when the professional tasked with the procedure is quite certain about where said electrodes are most likely to correctly identify the epileptogenic zone. Furthermore, it would be helpful to correctly deduce the location of the epileptogenic zone from the propagation zone, i.e., from the time evolution of a seizure through different areas of the mammalian brain. While the brain area from which a seizure originates can usually be positively detected through current imaging techniques, further improved, at least partially automated techniques for correctly identifying the epileptogenic zone would be appreciated by the clinician community.

It is one of the objectives of this application to outline methods and systems for estimating a location of an epileptogenic zone of a mammalian brain and thus increase the certainty with which SEEG electrodes may be optimally positioned.

SUMMARY

According to one aspect of the invention, a method for estimating a location of an epileptogenic zone of a mammalian brain may include receiving a structural skeleton model of a mammalian brain, wherein the structural skeleton model comprises a plurality of nodes and is based on non-invasive neuroimaging data and wherein connectivity information of the brain between different nodes is extracted from the non-invasive neuroimaging data.

Furthermore, a coupled brain network model is provided by populating each node of the structural skeleton model with a neural population model, wherein a neural population model corresponding to a node is coupled to further neural population models corresponding to further nodes according to the connectivity information extracted from the non-invasive neuroimaging data. After providing a first estimate of the location of the epileptogenic zone in the brain network model, wherein the first estimate of the location includes at least one of the plurality of nodes, a propagation zone is predicted in the brain network model based on an evolution of a simulated seizure starting from the first estimate of the location of the epileptogenic zone of the coupled brain network model. It is noted that any estimate of the location of the epileptogenic zone includes only a real subset of all the nodes of the brain network model. Furthermore, it is often assumed that the predicted propagation zone also does not include all nodes of the brain network model, but only includes a real subset of the nodes.

Further details of the method, the system, and a computer-readable medium containing instructions for executing such a method, such as hard drives, cloud-based storage systems, or portable storage media, e.g. CDs, DVDs or flash drives, are outlined in the detailed description below.

DETAILED DESCRIPTION

A structural skeleton model may be provided by importing structural and dMRI data of a mammalian brain via an input/output interface into a memory unit or a central processing unit of a computer system. Such data may include raw data, pre-processed data, or processed data. Raw structural or dMRI data of the patient's brain may be processed, for example, by using Scripts (https://github.com/timepx/scripts). Scripts makes use of various tools such as Freesurfer, FSL, MRTRIX3, and Remasher to reconstruct the individual cortical surface and large-scale connectivity of the mammalian brain. Structural and dMRI data may be used for building up a structural skeleton model specific to the particular patient's brain. Thus, the structural and dMRI data need to be taken from the specific brain, in which the epileptogenic zone is to be identified.

In many examples, a structural skeleton model derived from structural and dMRI data is parceled into a plurality of voxels, wherein a single node may be assigned to a single voxel or a plurality of voxels. A typical amount of nodes in a structural skeleton model of the human brain may add up to thousands of nodes. Each node may represent a volume of the mammalian brain either located on the cortical surface, within one of a plurality of cortical regions or within a plurality of subcortical regions. The parcellation may be performed, for example, using the Desikan-Killiany atlas. Using known techniques, tractography may be performed and a connectivity matrix connecting the different nodes of the structural skeleton model may be obtained, for example by summing track counts over each region of the parcellation and by normalizing the values so that the maximum value of the connectivity matrix is 1.

The method further provides a coupled brain network model such as a large-scale brain network model. These large-scale brain network models are known to provide insights into the mechanisms underlying the emergence of the resting-state network dynamics. In an example, a coupled brain network model is provided by populating each node of the structural skeleton model with a neural population model or neural mass model. In certain examples different nodes can be provided with different neural population models. However, in other examples all nodes of the structural skeleton model are populated with the same neural population model; furthermore different nodes may include different parameters for the neural population model. Neural population models are often represented by a system of coupled differential equations. One such example is the Epileptor as described, for example, in Jirsa et al., "On the nature of seizure dynamics", Brain, vol. 127, pages 2210-2230, which is referred to as Jirsa 2014 in the following and which is incorporated herein in its entirety. Each neural population model is coupled to further neural population models representing a different node in the structural skeleton model. The coupled neural population models are coupled so as to represent the connectivity information extracted from the non-invasive neuroimaging data. In other words, brain areas that according to the connectivity information are connected will be coupled in their respective neural population models whereas areas that according to the connectivity information are not connected will not be coupled in their respective neural population models. The neural population model may include state variables evolving over time and parameters, which govern the behaviour of the state variables over time. For example, a neural population model parameter may govern a degree of excitability of the model, i.e. an increase (or decrease) leads to a model more prone to exhibit autonomous seizure (or spiking) activity. Other parameters may govern the strength of a connection between two neural population models placed at different, yet connected nodes.

In certain examples of the method, the neural population model may include two or more coupled differential equations. In further examples, the system of coupled differential equations may include a fast and a slow subsystem. This means that the two differential equations operate on different time scales. A biological correlate of a fast subsystem may be local neuronal activity in the respective brain region. The slow subsystem may represent modulations of connections between different brain regions and their corresponding biological mechanisms such as neurotransmitter release, metabolic processes, or other, slower mechanisms forwarding signals through different brain networks.

Once the coupled brain network model is established, a programmer (inputting an estimated location of an epileptogenic zone by learned experience) or a computer system (inputting an estimated location of an epileptogenic zone based on algorithms) may provide a first estimate of the location of an assumed epileptogenic zone in the brain network model. Such an estimate may include at least one of the plurality of nodes. In certain examples, the neural population model is provided with a first set of parameters, the set of parameters known to be able to simulate or "trigger" a seizure. Triggering a seizure is, in some examples of the method, equivalent to a parameter set of a neural population model or a neural population model triggering seizures autonomously, i.e., the neural population model does not require external input into the neural population model to exhibit fast oscillations representing an ictal state occurring during a seizure. The nodes not identified as the first estimate of the location of the epileptogenic zone may be provided with a first set of parameters such that the neural population model does not trigger a seizure autonomously. In other words, the neural population models of areas in a non-epileptogenic zone require external input to exhibit fast oscillations representing an ictal state of a seizure. External input is understood as input from other nodes.

Based on the first estimate of the location of the epileptogenic zone and the time evolution of the neural population models of the nodes of the structural skeleton model a propagation zone may arise, i.e. a simulation of the brain network model may result in a propagation pattern of activity representing a location of a propagation zone in the brain network model. In other words, an epileptogenic zone exhibiting autonomous seizures may acquire further nodes through the coupling of the neural population models, which may show seizure activity in response to an autonomous seizure in the epileptogenic zone. Such a time evolution may be simulated by, for example, a system including a central processing unit, a memory unit, an input/output unit, or a computer-readable medium containing instructions for performing the method described above in connection with the system as outlined described above.

In a further embodiment, the method repeatedly estimates an epileptogenic zone and predicts a resulting propagation zone. If the resulting propagation zone does not match an observed propagation zone, or if a clinician, using his experience comes to the conclusion that the resulting propagation zone is unlikely, a new hypothesis of an epileptogenic zone is chosen and a new, re-sulting propagation zone is determined. Alternatively or additionally, instead of changing the location of the estimated epileptogenic zone, parameters of the neural population models may be changed. A change in the parameter values may lead to a different predicted propagation zone than the propagation zone determined using a first set of parameter values, and the new propagation zone may resemble previously recorded patient seizure or interictal data.

Specifically, in an embodiment of the method, the neural population model includes a parameter representing an excitability of the neural population model. Neural population models belonging to a node or a plurality of nodes of the epileptogenic zone may be assigned parameter values, such that the neural population models located in the estimated epileptogenic zone exhibit autonomous seizure activity. Depending on the complexity of the chosen neural population model, the model may also exhibit interictal and preictal activity. Other nodes are also assigned parameter values of the parameter representing a degree of excitability, however, the parameter values are chosen such that the neural model shows less excitability. In certain examples, the distribution of the parameter values is based on the distance from the epileptogenic zone. A distance can hereby be understood as a spatial distance or a time distance, i.e. the further away a node is from the epileptogenic zone, the lesser the excitability is chosen to be. Whether different nodes are connected may also influence the distribution of the parameter values of the parameter representing excitability.

Since the state variables of neural population models may represent biological parameters such as energy consumption, tissue oxygenation, or extracellular ion concentrations, or as previously mentioned a degree of excitability, the time evolution of simulation data gathered from the coupled brain network model may show a propagation zone that may or may not resemble seizure activity in a real patient, in particular in the patient from which the structural skeleton model was derived.

If the simulated propagation zone strongly deviates from a recorded propagation pattern of the patient, a further or new prospective epileptogenic zone can be chosen and a corresponding propagation zone can be predicted. An exemplary procedure is outlined in this application.

With the method outlined above, the probability of identifying the correct epileptogenic zone is highly increased in contrast to empirical or heuristic approaches. Once the structural skeleton model and the coupled brain network model have been established, a plurality of different epileptogenic zones can be tested and their corresponding propagation zones can be compared with previously or subsequently recorded real, brain seizure data.

In a further embodiment, a second estimate of the epileptogenic zone replaces the first estimate of the location of the epileptogenic zone if the simulated propagation zone of the first estimate differs from an observed propagation zone. Thus, without performing a surgical resection, a wrongly identified epileptogenic zone can be falsified by simulation. A simulated activity or signal may be compared to a real, recorded brain signal by standard statistical methods. If the simulated activity based on a first epileptogenic zone resembles the recorded signal well, or better than simulated activity from other epileptogenic zone estimates, the first epileptogenic zone can be kept and the other zones can be discarded.

In a further embodiment of the method, the propagation zone prediction may include the prediction of electrical activity data by employing a forward model. Forward models are used to map, for example, electrical activity or tissue oxygenation occurring within the brain volume at different times in different places to surface potentials, which may be measured by invasive or non-invasive surface electrodes during the recording of a SEEG, EEG or MEG. Since the analysis of SEEG and EEG data, in particular the comparison of different EEG and SEEG recordings, is easily accomplished by a plurality of commercial and non-commercial tools, simulated seizures can easily be compared to recorded seizure patterns. In particular, the implantation of electrodes can be postponed until a number of potential epileptogenic zones have been ruled out by the method as suggested in this application. Furthermore, the implantation scheme can be optimized with regard to best coverage and minimal invasiveness. This significantly improves the chances of performing a successful surgical resection.

In a further embodiment, the coupled brain network model may be further adapted by including a parameter representing a structural anomaly, such as an MRI lesion, in at least one node. For example, MRI lesions are known to have an effect on epileptic seizures and are a very good indicator for the location of the epileptogenic zone. However, the inclusion of MRI lesions in brain network models as described in this application has not previously been performed. An MRI lesion or other structural anomalies are important structural data further informing and improving the model. These data may be incorporated by local adjustments of parameters in the affected brain volume of the MRI lesion and will include manipulations of excitability and coupling. In recorded MRI data, an MRI lesion may be identified by characteristic dark patterns. While the structural skeleton model may result in showing that two nodes are connected with each other, an MRI lesion may add additional information to the brain network model in that the strength of the connection between nodes located in the area of the MRI lesions and nodes neighboring those nodes needs to be modified. By including MRI lesion information, the quality of the prediction and subsequent correct identification of the epileptogenic zone is greatly enhanced.

Depending on the location of the structural anomaly in the mammalian brain, the parameter values of the parameter representing the structural anomaly are distributed throughout the different nodes of the brain network model, such that the distribution resembles the occurrence of a structural anomaly in the mammalian brain.

In the following, exemplary implementations of the method will be described in detail.

Fig. 1      is a schematic overview of a system for estimating the location of an epileptogenic zone;

Fig. 2      illustrates the relation between different ways of understanding neural activity;

Figs. 3     are illustrations of a structural skeleton model and a coupled brain network model;

Figs. 4     show a flow chart of an exemplary method for finding an epileptogenic zone using the brain network model.

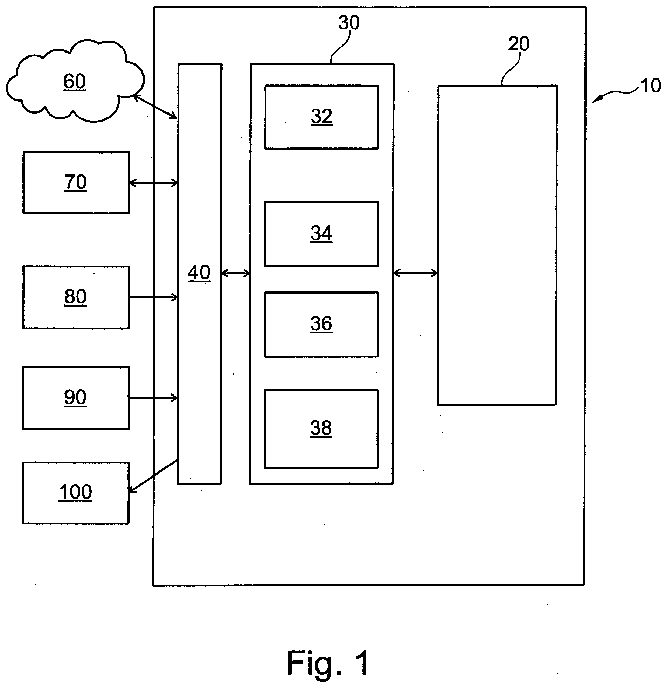

Fig. 1 shows a schematic overview of a system configured for estimating the epileptogenic zone in a patient. System 10 includes a central processing unit (CPU) 20, a memory unit 30, an input/output interface 40, and several input/output devices coupled to the interface 40.

The system can be a computer or a distributed system, in which the different units may include further components to function independent of each other, but are coupled via different data connections. The CPU may include a multiprocessor and/or multi-core processor for fast algorithm and data execution. The memory unit 30 may include primary storage linked to the CPU(s), random access memory (RAM), volatile memory as well as hard disks, flash memory units, or EEPROM units for storing data such as a structural skeleton 32, the coupled brain network model 34, various implementations of a neural population model 36 to placed in different nodes of the structural skeleton, and/or instructions 38 for executing different algorithms when processing and/or comparing the data. The input/output interface 40 can include several interfaces such as an input for a keyboard, a wired or wireless data connection for uploading or downloading data into the memory unit, or interfaces for connecting a display, such as a monitor, or a keyboard for inputting commands. As examples, the interface of Fig. 1 is coupled with the internet 60, a display 70, a keyboard 80 and a flash memory reader 90. Additionally, the interface 40 may be coupled to an EEG displaying unit 100.

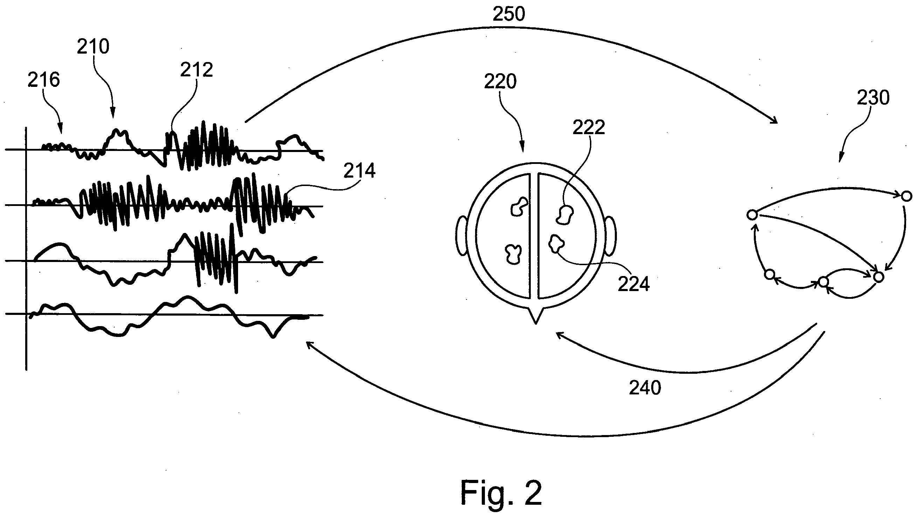

Fig. 2 illustrates how different brain signals can be recorded and how different imaging methods are connected to each other. Fig. 2 shows an exemplary scalp EEG recording 210 including different channels, which include different signals, such as ictal periods 212 or 214 and non-ictal or interictal period 216. Different channels correspond to different electrodes, which are located outside the human skull. EEG recordings have a high temporal resolution, but provide little information on the spatial distribution of recorded electrical signals throughout the brain. Neuroimaging methods, such as MRI (represented by longitudinal section through a skull 220) or methods based on MRI provide a much better spatial resolution. However, the temporal resolution of MRI based methods is limited as the signal recorded in MRI is not the electrical brain activity, but a signal representing the energy consumption. Modern techniques may link the seizure activity 212 or 214 to increased metabolic activity in area 222 or 224, respectively. MRI techniques include dMRI techniques, which allow a non-invasive extraction of connectivity information of a patient. Often the connectivity information is displayed in matrices to establish a time-space structure of the coupling (for further information, see for example Jirsa, V.K. "Neural field dynamics with local and global connectivity and time delay." Philos. Trans. A. Math. Phys. Eng. Sci. 367, 1131-1143, which is incorporated herewith in its entirety). The actual brain activity, which underlies the EEG recording 210 and MRI 220, is based on the electrical activity of different brain areas, which are connected with each other. The brain activity can be visualized by different nodes representing different areas of the brain, which are connected to each other according to the connectivity information. The resulting network (represented by reference numeral 230) displays localized, electric activity. The electric (and metabolic) energy needed for generating the electric brain activity can be seen in the MRI images as energy consumption. Furthermore, the localized, electric activity can be recorded by EEG electrodes. Techniques for mapping localized electric brain activity in different brain areas to EEG-like signals are generally known as forward models 240. Techniques for mapping EEG signals to localized, electric activity in different brain areas include inverse models 250 and usually much harder to describe than forward models or solutions (see for example, Jirsa, V.K. et al "Spatiotemporal forward solution of the EEG and MEG using network modeling", IEEE Trans. Med. Imaging 21, 493-504.).

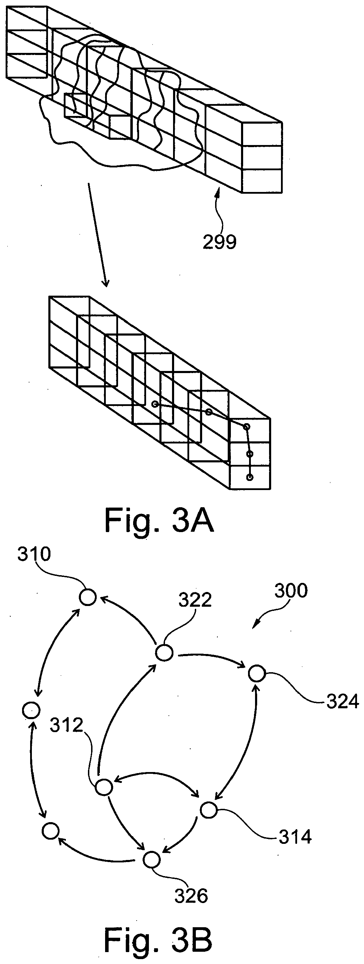

Fig. 3A and Fig. 3B illustrate the structural brain skeleton and the coupled brain network model. The structural brain skeleton of Fig. 3A is an individualized or patient-specific representation of large-scale brain connectivity. In other words, the structural skeleton model of the patient represents the patient's individual pattern of connections between different brain areas. Furthermore, the structural skeleton model may include information on the time it takes electric activity of one brain area to effect electric activity in another area. A structural skeleton often involves a parcellation of a patient's brain in voxels. The number of voxels (see an exemplary voxel 299) of a typical structural skeleton model may include between 100 and 100000 voxels, preferably between 5000 and 50000 voxels. The structural skeleton may include a representation of the patient's cortical surface only, or include further brain areas such as parts of the temporal gyrus, the frontal gyrus, the frontal lobe, the temporal pole, the occipital lobe, the parietal lobe. The dMRI data is used to extract the connections between different voxels of different brain areas.

Once the structural skeleton model has been completed, each voxel or in other embodiments only voxels, to which other voxels have a connection, are replaced by a node and the nodes may be populated by a neural network model. In many embodiments, the neural network model will include a system of coupled differential or difference equations.

In a preferred embodiment the neural network model used is the Epileptor as described in Jirsa 2014. The Epileptor includes five state variables acting on three different time scales. On the fastest time scale, state variables x1 and y1 account for fast discharges during a seizure. On the slowest time scale, the permittivity state variable z accounts for slow processes such as a variation in extracellular ion concentrations, energy consumption, and/or tissue oxygenation. The system exhibits fast oscillations during the ictal state through the variables x1 and y1. Autonomous switching between interictal and ictal states is realized via the permittivity variable z through saddle-node and homoclinic bifurcation mechanisms for the seizure onset and offset, respectively. The switching is accompanied by a direct current (DC) shift, which has been recorded *in vitro* and *in vivo* (see for example, Jirsa 2014). On the intermediate time scale, state variables and describe the spike-and-wave electrographic patterns observed during the seizure, as well as the interictal and preictal spikes when excited by the fastest system via the coupling. The Epileptor equations as outlined in Jirsa 2014, are repeated below:

$$\dot{x1} = y1 - f1(x1, y1) - z + I1$$

$$\dot{y1} = 1 - 5x1^2 - y1$$

$$\dot{z} = \frac{1}{\tau 0}(4(x1 - x0) - z)$$

$$\dot{x2} = -y2 + x2 - x2^3 + I2 + 0.0002g(x1) - 0.3(z - 3.5)$$

$$\dot{y2} = \frac{1}{\tau 2}(-y2 + f2(x1, x2))$$

where

$$f1(x1, x2) = \begin{cases} x1^3 - 3x1^2 & if\ x1 < 0 \\ (x2 - 0.6(z-4)^2)x1 & if\ x1 \geq 0 \end{cases}$$

$$f2(x1, x2) = \begin{cases} 0 & if\ x2 < 0 \\ 6(x2 + 0.25)x1 & if\ x2 \geq 0 \end{cases}$$

$$g(x1) = \int_{t0}^{t} e^{-\gamma(t-\tau)}x1(\tau)d\tau$$

and $xo = -1.6$; $\tau 0 = 2857$; $\tau 2 = 10$; $I1 = 3.1$; $I2 = 0.45$; $\gamma = 0.01$. The parameter x0 controls the tissue excitability, and is epileptogenic, i.e. is triggering seizures autonomously for a critical value $x0 > -2.05$, otherwise the tissue is healthy.

Due to the time scale separation, the five dimensional epileptor can be reduced to a two-dimensional system:

$$\begin{cases} \dot{x1} = -x1^3 - 2x1^2 + 1 - z + I1 \\ \dot{z} = \frac{1}{\tau 0}(4(x1 - x0) - z) \end{cases}$$

With $\tau 0 = 2857$; *and I*1 = 3.1. In other embodiments other time-scale separated differential systems than the Epileptor may be used, wherein the fast variable of the neural network model represents fast discharges, and wherein the slow variable represents the switching between ictal and interictal states through a bifurcation of the dynamic system of the neural network model.

The different nodes of the brain network model are coupled by permittivity coupling, i.e. the neural network models of connected nodes are coupled in their slow variables. A node i is coupled to a remote node j by introducing a coupling term $K_i = \sum_{j=1}^{N} K_{ij}(x1_j - x1_i)$ to the permittivity state variable of the neural network model of node i. $K_{ij}$ includes the connectome $C_{ij}$ (a value representing the connectivity between different voxels or nodes), and a scaling factor G The index j runs over all nodes connected to node i. The equation fort he slow variable then reads, for example

$$\dot{z}_i = \frac{1}{\tau 0}\left(4(x1_i - x0_i) - z_i - \sum_{j=1}^{N} K_{ij}(x1_j - x1_i)\right)$$

The permittivity coupling from node i to node j can also be chosen to include a signal transmission delay to account for real brain transmission delays. The delay is introduced by modifying the coupling term such that the input from node j to node i is delayed by a time $t_{delay}$, i.e. $K_{ij}(x1_i(t)-x1_j(t-t_{delay}))$. After all nodes of the structural skeleton are populated by a neural network model, such as the Epileptor outlined above, the brain network model can be used for estimating the location of an epileptogenic zone using the brain network model.

To define a node as belonging to an epileptogenic zone, said nodes neural network model, i.e. the Epileptor's *x*O value of said node is set to a value greater than -2.05, i.e. is set to a value for which the neural network model shows autonomous triggering of seizures (even an uncoupled or isolated Epileptor with a value of *G* = O). Alternatively a new variable $\Delta x0=x0+2.05$ may be introduced. When providing a first estimate of an epileptogenic zone, the neural network models of a first number of nodes are provided with a parameter set, which allows the neural network models to exhibit autonomous seizures. The parameter set may include values for $K_{ij}$, I1, I2, x0 and optionally others.

Fitting parameters of the neural population models of the brain network model

After the location of the epileptogenic zone has been estimated, it may be advantageous to alter the distributions of parameter values of different parameters of the neural population models based on the distance of a node from the epileptogenic zone. For example, the parameter values of a parameter representing excitability, such as x0, can be distributed by a Gaussian distribution, i.e. the parameter values being highest in the epileptogenic zone and being smaller the further away the corresponding node is from the epileptogenic zone. Changing the distribution scheme may consequently result in different propagation zones. Distance between different nodes can also be based on the strength of connections between different nodes in the brain network model.

In the following, the fitting of parameter values for nodes of the brain network model is further explained in the case of empirical, i.e. recorded SEEG patient data.

Obtaining estimates of the parameters of the network model, given the available functional data can be performed within a Bayesian framework using a reduced Epileptor model (see Proix et al "Permittivity Coupling across Brain Regions Determines Seizure Recruitment in Partial Epilepsy" J. Neurosci. 34, 15009-15021, which is incorporated herein in its entirety) and a reduced functional data set for the fitting. In the following, an exemplary data fitting method is provided for SEEG data.

The SEEG data are windowed and Fourier transformed to obtain estimates of their spectral density over time. Then SEEG power above 10 Hz is summed to capture the temporal variation of the fast activity. These time series are corrected to a pre-ictal baseline, log-transformed and linearly detrended over the time window encompassing the seizure. Contacts are selected, which present greater high-frequency activity than their neighbors on the same electrode. Given that, contrary to M/EEG, the SEEG lead field is very sparse, three nodes per contact are used in the network model. Other nodes are not recruited and rest at their fixed points. The effect is approximated by the fitting through a constant sum over the corresponding elements of the structural connectivity matrix. Next, one may use an observation model that incorporates an SEEG forward model, under the assumption that the variable describes fluctuations in the log power of high frequency activity, predicting sensor log power, with normally distributed observation error.

Uninformative priors are placed on the hidden states' initial conditions, while their evolution follows an Euler-Maruyama discretization of the corresponding stochastic differential equations with linear additive normally distributed noise. Uninformative priors are also placed on the excitability parameter per node, observation baseline power, scale and noise. Finally, the length of the seizure is also allowed to freely vary to match that of a given recorded seizure. Structural connectivity specifies a prior on the connectivity used in the generative method. This model is implemented using Stan, a software for Bayesian inference, which implements both Hamiltonian Monte-Carlo and automatic variational inference algorithms for generic differential probability models (see Hoffman, M.D., Gelman, A. "The No-U-Turn Sampler: Adaptively Setting Path Lengths in Hamiltonian Monte Carlo" arXiv Prepr. 1-30; The Stan Development Team "A C++ library for Probability and Sampling"). This approach takes advantage of the efficiency of the variational algorithm, which constructs an approximate proxy distribution on the true posterior optimized via stochastic gradient ascent.

To simulate the system of stochastic differential equations, an Euler-Maruyama integration scheme with an exemplary integration step of 0.05 may be used. Additive white Gaussian noise is introduced in the variables and with mean 0 and variance 0.0025 (see Jirsa 2014). Other variables experienced only little or no noise due to their high sensitivity. 256 time steps are equivalent to one second of real time to obtain realistic frequency ranges, seizure lengths, and matched intracranial EEG sampling frequency. Finally, for the stimulated seizure, a rectangular function in time was applied on the z variable of the stimulated region (amplitude: 0.5, length: 2 s).

The result of the above fitting procedure is a parameter value for each node, thereby establishing a distribution (spatial, temporal or spatio-temporal) for each node of the brain network model). A map of the distribution may also be referred to as a heat map.

The choice of the first estimate of an epileptogenic zone may be provided by a clinician, followed by an iterative process between clinician and the method disclosed herein or a fully automated approach, resulting in a refinement of the epileptogenic zone via a second, third or fourth estimate, which may be subsequently provided; however, the systems and methods as disclosed in this application may also include an automated choice simulation of different first, second and further epileptogenic zones to arrive at a preferred epileptogenic zone, the propagation zone, of which resembles previously recorded brain activity best.

The relationship between the brain network model, an estimate of an epileptogenic zone, a propagation zone, and other regions is illustrated in Fig. 3B. The (simplified due to a reduced number of nodes) brain network model 300 includes a plurality of nodes 310. Each node includes a neural network model such as the epileptor. The different nodes are coupled to further nodes according to the connectivity information, for example the connectome $C_{ij}$. The coupling between nodes i and j can be uni- ($C_{ij} \neq 0$, $C_{ij} = 0$) or bi-directional ($C_{ij} \neq 0, C_{ij} \neq 0$), represented by uni-directional or bi-directional arrows, respectively. An epileptogenic zone is first estimated to include two nodes 312 and 314, i.e. the neural network models of each node of the estimated epileptogenic zone can exhibit autonomous seizures. The resulting propagation zone includes nodes which are coupled to the nodes 312 and 314, for example nodes 322, 324 and 326. The nodes of the propagation zone, while not able to exhibit seizures autonomously, are captured by the nodes of the epileptogenic zone, i.e. receive input from the nodes of the epileptogenic zone through the permittivity coupling and are driven to exhibit seizure behavior as well. The remaining nodes of the brain network are not captured by the epileptogenic zone in the sense that the nodes do not receive sufficient input from other nodes to be driven into a seizure state.

While the connectivity information derived from the dMRI procedure may be sufficient, the applicants have found that use of further information into the brain network model improves the ability of the systems and methods to find the location of the epileptogenic zone. Further information may include structural anomalies. For example, an MRI lesion may be included by modifying the local connectivity of the area involved. This may be achieved by changing the scalar factor G to $G_{local}$, with $G < G_{local}$, to represent the structural anomaly. The local connectivity topology of the area, i.e. the nodes affected by the structural anomaly, is not changed but scaled up by the factor $G_{local}$. Such anomalies which may be modeled can include pachygaria, hamartoma and others.

When using the brain network model to find the propagation zone of an estimate of an epileptogenic zone, it has proven helpful, to translate the brain network model activity, using a forward model, into SEEG, fMRI, or EEG activity. Since SEEG, fMRI and EEG data (i.e. real, not simulated patient data) can be easily obtained from a patient, the translated brain network model activity can be easily compared to real EEG patient data to access whether the estimate of the epileptogenic zone provides brain activity which is similar to measured brain data, or whether a new epileptogenic zone must be chosen to arrive at a better fit between the simulated brain network model activity and the measured brain data.

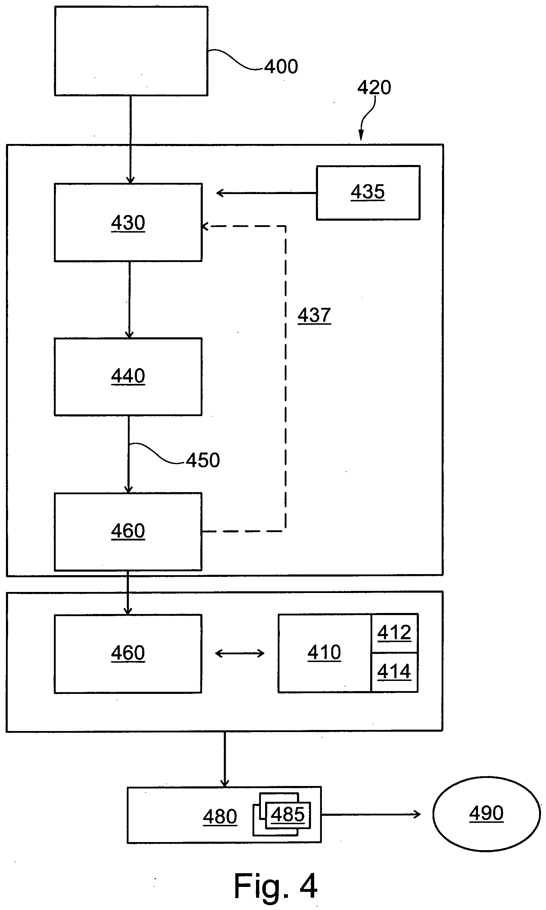

The systems and methods discussed in this application may also encompass the uploading, reception or generation of a patient's brain network model based on a patient's structural skeleton model as indicated in Fig. 4. Once a patient's brain network model 400 has been generated or uploaded into a system such as the system of Fig. 1 and real brain data 410 (such as EEG 412, fMRI 414, SPECT and/or SEEG data), from the patient including a period of a simple seizure (i.e. a seizure not capturing a propagation zone) or a complex seizure (i.e. a seizure capturing a propagation zone) has been generated or uploaded to the system, an estimation method 420 is initiated which includes: choosing a first estimate of an epileptogenic zone 430; a subsequent simulation of a resulting propagation zone 440 and an optional translation 450 via a forward model into simulated EEG, fMRI, SPECT or SEEG activity 460; a comparison (step 470)between the measured, real brain data 410 and the simulated activity 460. The estimation method may optionally include using prior information 435 to obtain an estimate of a first epileptogenic zone including at least one node such as a clinician's estimate of the first epileptogenic zone or an automated starting choice. Furthermore the epileptogenic zone may be varied after generating the simulated activity 460 for a specific epileptogenic zone and steps 430 through 470 may be repeated with a new estimate of a location of the epileptogenic zone. The varied epileptogenic zone estimate may include neighboring nodes, additional nodes, or a subset of nodes from the previous or first estimate of the epileptogenic zone. The estimation method may then include a decision engine 480 (applying statistical methods as known in the prior art to compare time-series; the decision engine may be a software module for deciding which simulated time-series fits the real data best) to find the best match 485 between simulated brain network model activity and real brain data and output the epileptogenic zone of the best matching data 490 as a suggestion for a brain area of surgical resection or a suggestion for a pre-surgical implantation site of electrodes for recording SEEG data to minimize the traumatic impact for a patient.

In the following the process of arriving at an epileptogenic zone estimate is summarized:

a) A clinician obtains non-invasive brain images of the patient including MRI, DTI, EEG, MEG. Using traditional approaches (standard-of-care) the clinician interprets all data and makes his/her initial hypothesis on the location of the EZ and a proposal of an implantation scheme for the SEEG electrodes.

b) A first virtual patient brain model (i.e. brain network model) is constructed, and optionally uploaded to a different computer system and the Clinician's first hypothesis on EZ is used as a first estimate of the location of the epileptogenic zone. Subsequently, the computer system generates simulated imaging data (EEG, MEG), which will be analysed using the clinical standard visualization (visual inspection of EEG time series by expert eye) and analysis tools (biomarkers such as Functional Connectivity, Epileptogenicity index, H2). Through adjustment of EZ hypothesis (i.e. by changing the spatial distribution of excitability values, as well as including MRI lesion information in the brain network model) the clinician will attempt to converge model behavior with empirical patient data. This iterative process is close to the spirit of contemporary patient management staff meetings in hospitals, where individual patient cases are discussed.

c) Relying on machine learning methods, model parameters in the brain network model will be fitted automatically against empirical EEG/MEG data.

d) Level 1b) and c) tasks will provide an updated hypothesis on the EZ. Once the updated location of the epileptogenic zone is deemed sufficient, the computer system generates forward solutions of SEEG data and proposes an optimal implantation scheme for SEEG electrodes.

The method outlined by Fig. 4 and its corresponding description can be performed using non-invasive real brain data, such as EEG data, or invasive real brain data, such as SEEG data. This leads to a further embodiment of the method, where in a first stage a location of the epileptogenic zone is estimated based on non-invasive patient data, the resulting estimate of the epileptogenic zone is used as a suggested location for performing an implantation of electrodes for recording SEEG data and further refining the method, and this the estimated location of the epileptogenic zone based on the invasive real brain data, providing as an output an even better estimate of the epileptogenic zone. This epileptogenic zone can than be used as a candidate for surgical resection.

In the following, an exemplary implementation of a method embodiment: A patient was diagnosed with bi-temporal epilepsy and experienced simple and secondary generalized seizures, which were accompanied by déjà-vu hallucinations, associated with palpitations, horripilation, and frisson sensations. Fixed gaze, chewing up and pallor were observed during the seizure. In the post-critic period the patient showed temporal disorientation, repetition of the same questions and retrograde amnesia during one week. The MRI examination revealed a hypothalamic hamartoma. Surface EEG recordings revealed interictal spikes and indicated a bias towards the left hemisphere. Based on the presurgical evaluation, seven SEEG electrodes were implanted in the left hemisphere, and two in the right hemisphere. One electrode was implanted in the hypothalamic hamartoma. During two weeks of continuous SEEG recordings, 6 simple seizures localized in the right hippocampus, and two complex seizures starting in the right hippocampus and then recruiting the left hippocampus, the left temporal lobe and the hypothalamic hamartoma were recorded.

The large-scale connectivity of the patient was reconstructed, in particular the weight and tract length matrices generating a structural skeleton model. The tract length matrix divided by the signal transmission speed defines the time delays, thereby establishing the space-time structure of the coupling and allowing a full virtualization of the patient's brain model. A hypothalamic hamartoma was included in the model by changing its effect on local connectivity through factor $K_{ij}$.

To generate a brain network model for the patient, each voxel of the structural skeleton was replaced by a node. Each node of the resulting network was set with an epileptor as described above. The nodes were connected via permittivity coupling, which acting on a slow time scale and allowing the spread of the seizure though the network by recruiting regions not in the epileptogenic zone. Each node was set with a different excitability parameter x0. The value of the excitability was set heterogeneously across the network: The epileptogenic zone was set with values of excitability for initiating autonomous triggering of seizures, the propagation zone was set with values of excitability below a value for autonomous triggering of seizures, however, the values of excitability were higher than in areas which were not considered to be part of the propagation zone. A systematic parameter space exploration was performed by varying the following parameters: (i) the global coupling strength G, which is a scalar factor multiplying the whole connectivity matrix, (ii) the local coupling strength G_hyp of the hypothalamus, which is a scalar factor multiplying the contribution of the hypothalamus to the connectivity matrix, (iii) the excitability values of the right hippocampus, (iv) the excitability values of the regions not recruited in the propagation zone (other regions). The excitability values of the other regions in the epileptogenic zone and the propagation zone were fixed (see Table 1). To characterize the four-dimensional parameter space, we define quantities relevant for seizure description such as (i) regions involved in the seizure, (ii) seizure length, (iii) length of time delays before recruitment of other regions, (iv) seizure frequency in each region.

Table 1:

| Name of the region | x0+2.5 | Zones |
|---|---|---|
| Right hippocampus | 1.3 | epileptogenic zone |
| Left hippocampus | 0.4 | epileptogenic zone |
| Left hypothalamus | 0.4 | epileptogenic zone |
| Right hypothalamus | 0.4 | epileptogenic zone |
| Brain Stem | 0.31 | propagation zone |
| Left parahippocampal | 0.27 | propagation zone |
| Left thalamus | 0.24 | propagation zone |
| Left temporal pole | 0.16 | propagation zone |
| Other regions | -0.2 | Other regions |

A representative set of parameters (G=10; G_hyp=10) matching the patient's seizure with regard to these seizure description quantities was selected. The brain network model was used for simulations over a period of 20 seizures and the forward solution for the SEEG electrodes was computed. Simple seizures and complex seizures were generated with similar regions recruited compared to the real SEEG recordings. The left hippocampus was stimulated and a propagation pattern in the left temporal lobe, similar to the SEEG recordings, was observed.

To compare the efficacy of the method, the results of the simulations, a clinician's prediction was compared to results won from simulations of the brain network model. Comparing the clinician's prediction and the simulated epileptogenic and propagation zone's excitability, the applicant has found a significant overlap between the clinician's prediction and the simulation results, demonstrating the applicability of the approach outlined in this application. When running further simulations and in particular converting the brain network model's activity as EEG data (via a forward model), the simulated EEG data shows a strong similarity with recorded EEG data. The spatial distribution of the degree of excitability is based on distance of an area from the epileptogenic zone. In a similar fashion, a parameter value distribution for a parameter representing an MRI lesion can also be included in the brain network model.

As the simulation of the brain network model can be very time-intensive, methods for simplifying the calculations without loosing too much of the brain network model's dynamic behaviour will be discussed in the following.

By taking advantage of the slow-fast dynamics of the two-dimensional Epileptor, averaging methods are applied by reducing the system to its slow dynamics. This approximation holds as long as $\tau_0 \gg 1$. The two-dimensional system is then reduced to a one-dimensional system, since $x_i = F(z_i)$. Thus, the 2N dimensional system (where N is the number of nodes in the brain network model) becomes 1N dimensional, and is expressed as:

$$\dot{z} = \frac{1}{\tau_0}\left(-4x_0 + 4F(z_i) - z - \sum_{i=1}^{N} K_{ij}(F(z_j) - F(z_i))\right)$$

Computing $F(z_i)$ explicitly by approximating the third order polynomial in x with a second order polynomial by doing a second order Taylor expansion in x at -4/3, results in $\dot{x}_i \approx 2x_i^2 + 16/3\,x_i + 4.1 + 64/27$, which leads to the expression $F(z_i) = 1/4\left(-16/3 - \sqrt{8z_i - 629.6/27}\right)$. The coupling term $K_{ij}(x_j - x_i)$ is weak compared to the other terms of the 1N dimensional system, and in particular the term in $x_0$. The difference coupling leads to weak coupling terms in a linear stability analysis. The weakness depends on the global coupling strength factor G and the normalization of the connectivity matrix, however one can show that this approximation holds over a larger range than for other neural network models.

For a generic model with weak coupling, at the linearization point at which the linear stability analysis is performed, the leading eigenvectors are derived and the eigenvectors only depend on the topological connection to the epileptogenic zone.

Considering the generic model:

$$\dot{Z}_i = G(Z_i) + X_{0,i} + \sum_{j=1}^{N} K_{ij} H(Z_j)$$

With $\left| \sum_{j=1}^{N} K_{ij} H(Z_j) \right| \ll 1$. The fix point solution is given in a first order approximation by:

$$\overline{Z}_i = G^{-1}(-X_{0,i}),$$

which is the fixed point of the uncoupled system. We assume the leading eigenvector will be small at the first order for all components except for the epileptogenic node, whose coordinate is arbitrarily set to $v_1 = 1$. Computing the Jacobian and writing the system for the leading eigenvector gives:

$$(G'(G^{-1}(-X_{0,i})) - \lambda_1)v_i + \sum_{j=1}^{N} K_{ij} H'(G^{-1}(-X_{0,i}))v_j = 0$$

In the above equation, each term in the sum is of order 2, except for the term in $v_1$ and since $v_1 = 1$, one finds $\lambda_1 = G'(G^{-1}(-X_{0,1}))$. All the other terms are calculated iteratively, finding

$$v_i = -\frac{K_{i1}}{G'(G^{-1}(-X_{0,i})) - G'(G^{-1}(-X_{0,1}))}.$$

In particular for the epileptor, $G(Z_i)=4F(Z_i)-Z$, and

$$G'(Z_i) = 4F'(Z_i) - 1 = \frac{-1}{\sqrt{8Z_i + 629.6/27}} - 1.$$

Checking the validity of the above analytical expression numerically can be performed by computing the full system for the same connectivity matrix and the reduced system with numerical computation of the Jacobian and the analytical expression. A check reveals that the components (other than $v_1$) are of second order. A consequence for real connectivity matrices is that, since only some connections are of important weight for each node, these regions will systematically come more often in the prediction.

The linearization procedure can be easily implemented in a set of instructions for running simulations of a linearized brain network model. Since linear problems can be much more easily be computed than non-linear problems, the simulation time can be greatly reduced.

**Claims**

1. Method for estimating a location of an epileptogenic zone of a mammalian brain, the method including the following steps:

   a) Receiving a structural skeleton model of a mammalian brain, wherein the structural skeleton model comprises a plurality of nodes and is based on non-invasive neuroimaging data and wherein connectivity information of the brain between different nodes is extracted from the non-invasive neuroimaging data;

b) Providing a coupled brain network model by populating each node of the structural skeleton model with a neural population model, wherein the neural population model corresponding to a node is coupled to further neural population models corresponding to further nodes according to the connectivity information;

c) Providing a first estimate of the location of the epileptogenic zone in the brain network model, the first estimate of the location including at least one of the plurality of nodes;

d) Predicting a location of a propagation zone in the brain network model based on the first estimate of the location of the epileptogenic zone by at least one simulation of the coupled brain network model.

2. Method according to claim 1, wherein a second estimate of the epileptogenic zone replaces the first estimate of the location of the epileptogenic zone, if the simulated propagation zone of the first estimate differs from an observed propagation zone.

3. Method according to claim 2, wherein further estimates of the epileptogenic zone replace the second estimate, if the simulated propagation zone of the second estimate differs from an observed propagation zone.

4. Method according to any of the previous claims, wherein the steps of providing an estimate of an epileptogenic zone and predicting a propagation zone are iteratively repeated, wherein the location of the epileptogenic zone is changed and/or wherein parameters of the neural population model are changed.

5. Method according to any of the previous claims, wherein the neural population model includes a parameter representing an excitability of the population model and assigning parameter values indicating a first degree of excitability to the at least one of the plurality of nodes of the epileptogenic zone, and assigning parameter values indicating a lower than the first degree of excitability to nodes coupled to nodes of the epileptogenic zone.

6. Method according to claim 5, wherein a spatial distribution of the parameter values indicating the degree of excitability throughout the brain network model is based on a distance of a node from the epileptogenic zone.

7. Method according to any of the previous claims, wherein the coupled brain network model includes a representation of a structural anomaly, preferably an MRI lesion, in at least one node.

8. Method according to claim 7, wherein the neural population model includes a parameter indicating a degree of the structural anomaly.

9. Method according to any of the previous claims, wherein the neural population model is represented by at least a first and a second differential equation and a time scale of the first differential equation is faster than a time scale of the second differential equation.

10. Method according to claim 9, wherein different nodes are coupled via the second differential equation of the respective nodes.

11. Method according to any of the previous claims, wherein the propagation zone prediction includes a prediction of electric activity data.

12. Method according to any of the previous claims, wherein the method includes a forward model for mapping brain data to electroencephalogram data, and data representing the propagation zone is fed to the forward model.

13. Method according to any of the previous claims, wherein a location for implanting stereotactic electrodes in the mammalian brain is based on an epileptogenic zone estimation.

14. Method according to any of the previous claims, wherein a coupling between the at least one node of the epileptogenic zone and a node coupled to the at least one node is changed in a simulation and the thereby changed brain network model is used for predicting an alternative propagation zone.

15. System including a central processing unit, a memory unit and an input/output interface, the device configured for estimating the location of an epileptogetic zone of a mammalian brain including the following steps:

a) Loading a structural skeleton model of a mammalian brain in the memory unit, wherein the structural skeleton model comprises a plurality of nodes and is based on non-invasive neuroimaging data and wherein connectivity information of the brain between different nodes is extracted from the non-invasive neuroimaging data;

b) Loading a coupled brain network model in the memory unit, wherein each node of the structural skeleton model comprises a neural population model and wherein the neural population model corresponding to a node is coupled to further neural population models corresponding to further nodes according to the connectivity information;

c) Inputting a set of starting parameters for providing a first estimate of the location of the epileptogenic zone in the brain network model, the first estimate of the location including at least one of the plurality of nodes;

d) Evolving, by the central processing unit, and storing a location of a propagation zone in the brain network model based on the first estimate of the location of the epileptogenic zone by at least one simulation of the coupled brain network model.

16. System according to claim 15, wherein the Evolution of the propagation zone includes a time-dependent evolution of the neural population models of the plurality of nodes.

17. System according to any of claim 15 or 16, wherein the device is further configured for comparing a stored propagation zone with a recorded propagation signal of the brain.

18. System according to claim 17, wherein the device further includes a decision engine for determining a validity of the estimated epileptogenic zone.

19. System according to any of claims 15 through 18, wherein the device further includes a lesion engine for modifying the connectivity information to simulate lesion effects.

20. Computer-readable medium including a set of instructions for executing the method of any of claims 1 through 14.

21. Method for adjusting parameters of a brain network model used for estimating a location of an epileptogenic zone of a mammalian brain, wherein the brain network model includes a plurality of nodes and a neural population model is placed in each node, the method including the following steps:

a) Distributing parameter values of a parameter indicating a degree of excitability of the neural population model over the plurality of nodes, the distribution based at least in part on the distance of a node to a node of the epileptogenic zone estimate;

b) Distributing parameter values of a parameter indicating a structural anomaly over the plurality of nodes based on a location of a structural anomaly derived from non-invasive neuroimaging data;

c) Fitting the parameter values of the parameter indicating the degree of excitability and/or the parameter indicating the structural anomaly, such that simulation data of the brain network model is fitted to recorded patient data, wherein the fitting is based on a clinician's input or an automatic fitting procedure;

d) Changing the location of the estimated epileptogenic zone in the brain network model and comparing simulation data of the brain network model including the changed location and recorded patient's data.

Fig. 1

Fig. 2

Fig. 3A

Fig. 3B

Fig. 4

Europäisches
Patentamt
European
Patent Office
Office européen
des brevets

## EUROPEAN SEARCH REPORT

Application Number

EP 16 16 6489

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FRANCES HUTCHINGS ET AL: "Predicting Surgery Targets in Temporal Lobe Epilepsy through Structural Connectome Based Simulations", PLOS COMPUTATIONAL BIOLOGY, vol. 11, no. 12, 10 December 2015 (2015-12-10), page e1004642, XP055345338, US ISSN: 1553-734X, DOI: 10.1371/journal.pcbi.1004642 * abstract * * section 'Introduction' * * section 'Results' * * secton 'Methods' * * figures 1-7 * | 1-15 | INV. A61B5/055 A61B5/00 G06F19/00 |
| A | SANZ-LEON PAULA ET AL: "Mathematical framework for large-scale brain network modeling in The Virtual Brain", NEUROIMAGE, vol. 111, 13 January 2015 (2015-01-13), pages 385-430, XP029120616, ISSN: 1053-8119, DOI: 10.1016/J.NEUROIMAGE.2015.01.002 * section 'Introduction' * * section 'Results' * * section 'Discussion' * | 1-15 | TECHNICAL FIELDS SEARCHED (IPC) A61B G06F |
| A,D | JIRSA ET AL.: "On the nature of seizure dynamics", BRAIN, vol. 127, 11 June 2014 (2014-06-11), pages 2210-2230, XP002767233, * abstract * * page 2213 - page 2226 * * figures 1-8 * | 1-15 | |

-/--

The present search report has been drawn up for all claims

| Place of search | Date of completion of the search | Examiner |
|---|---|---|
| The Hague | 16 February 2017 | Marteau, Frédéric |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt

European
Patent Office

Office européen
des brevets

**EUROPEAN SEARCH REPORT**

Application Number

EP 16 16 6489

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SEBASTIEN NAZE ET AL: "Computational Modeling of Seizure Dynamics Using Coupled Neuronal Networks: Factors Shaping Epileptiform Activity", PLOS COMPUTATIONAL BIOLOGY, vol. 11, no. 5, 13 May 2015 (2015-05-13), page e1004209, XP055345366, US ISSN: 1553-734X, DOI: 10.1371/journal.pcbi.1004209 * abstract * | 1-15 | |

----- 

TECHNICAL FIELDS
SEARCHED       (IPC)

The present search report has been drawn up for all claims

| Place of search | Date of completion of the search | Examiner |
|---|---|---|
| The Hague | 16 February 2017 | Marteau, Frédéric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JIRSA et al.** On the nature of seizure dynamics. *Brain,* vol. 127, 2210-2230
- **JIRSA, V.K.** Neural field dynamics with local and global connectivity and time delay. *Philos. Trans. A. Math. Phys. Eng. Sci.,* vol. 367, 1131-1143
- **JIRSA, V.K. et al.** Spatiotemporal forward solution of the EEG and MEG using network modeling. *IEEE Trans. Med. Imaging,* vol. 21, 493-504
- **PROIX et al.** Permittivity Coupling across Brain Regions Determines Seizure Recruitment in Partial Epilepsy. *J. Neurosci.,* vol. 34, 15009-15021